(19) Europäisches Patentamt
European Patent Office
Office européen des brevets

(11) Publication number: **0 315 434 A2**

# (12) EUROPEAN PATENT APPLICATION

(21) Application number: **88310314.5**

(22) Date of filing: **02.11.88**

(51) Int. Cl.[4]: **C 07 D 213/65**
C 07 D 401/12,
C 07 D 213/81, A 61 K 31/44,
A 61 K 31/50, A 61 K 31/505

(30) Priority: **02.11.87 IL 84331**

(43) Date of publication of application:
**10.05.89 Bulletin 89/19**

(84) Designated Contracting States:
**AT BE CH DE ES FR GB GR IT LI LU NL SE**

(71) Applicant: **YISSUM RESEARCH AND DEVELOPMENT COMPANY OF THE HEBREW UNIVERSITY OF JERUSALEM**
**46, Jabotinsky Street P.O. Box 4279**
**Jerusalem 91042 (IL)**

(72) Inventor: **Sarel, Shalom, Prof.**
**Jabotinsky 36**
**Jerusalem (IL)**

**Avramovici-Grisaru, Shelly**
**Tchernokovsky 29**
**Jerusalem (IL)**

**Hershko, Chaim**
**Shachrai 18**
**Jerusalem (IL)**

**Link, Gabriella**
**Chevroni 116/42**
**Jerusalem (IL)**

**Spira, Dan**
**Bialik 14**
**Jerusalem (IL)**

(74) Representative: **Bizley, Richard Edward et al**
**BOULT, WADE & TENNANT 27 Furnival Street**
**London EC4A 1PQ (GB)**

## (54) Pyridoxal hydrazone derivatives their production and use and pharmaceutical compositions containing the same.

(57) A pyridoxal hydrazone derivative of the general formula I

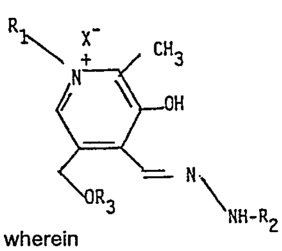

wherein
$R_1$ is an H atom or an alkyl, a methoxy-carbonylmethyl group or ethoxy-carbonylmethyl group
X is a halogen or hydroxyl group and
$R_2$ is a 4, 5, 6 or 7 membered heterocycle having one or more nitrogen, sulfur, or oxygen atoms in the heterocyclic ring, said ring being optionally substituted by one or more oxo, halogen, hydroxy, nitro, amino, cyano, trifluoromethyl, lower alkyl or alkoxy groups, and $R_3$ is a hydrogen atom or an acetyl, propionyl or succinyl group and pharmaceutical compositions containing such derivatives.

EP 0 315 434 A2

## Description

# PYRIDOXAL HYDRAZONE DERIVATIVES THEIR PRODUCTION AND USE AND PHARMACEUTICAL COMPOSITIONS CONTAINING THE SAME

The invention provides a pyridoxal hydrazone derivative of the general formula I

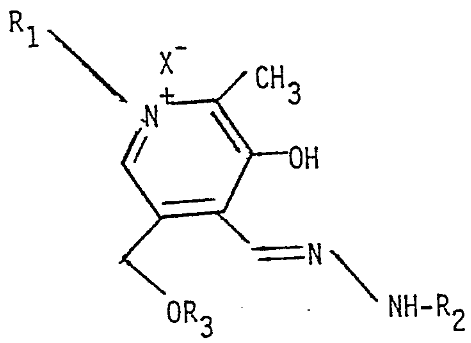

wherein

$R_1$ is an H atom or an alkyl, a methoxy-carbonylmethyl group or ethoxy carbonylmethyl group

X is a halogen or hydroxyl group and

$R_2$ is a 4, 5, 6 or 7 membered heterocycle having one or more nitrogen, sulfur, or oxygen atoms in the heterocyclic ring, said ring being optionally substituted by one or more oxo, halogen, hydroxy, nitro, amino, cyano, trifluoromethyl, lower alkyl or alkoxy groups, and $R_3$ is a hydrogen atom or an acetyl,propionyl or succinyl group and pharmaceutical compositions containing these derivatives.

The present invention relates to pyridoxal hydrazone derivatives and to pharmaceutical compositions containing the same.

More particularly the present invention relates to the discovery of a family of pyridoxal hydrazone derivatives which can be used in pharmaceutical compositions to treat iron overload in mammals by in vivo iron chelation and which are also useful for the prophylactic or the therapeutic treatment of malaria, as well as for the therapeutic treatment of hepatoma, melanoma and carcinoma.

There are three important pathologic states in which iron overload causes massive deposition of the metal in the parenchyma of a number of organs, causing tissue damage and ultimately proving fatal. These are: (i) idiopathio haemochromatosis, (ii) Bantu siderosis;and (iii) transfusional siderosis.

In the first two cases, overload is consequent to excessive adsorption and is therefore most easily treated by withdrawal of blood from a vein (phlebotomy). In fact, 250 mg of iron is removed for every 500 ml of whole blood. In the case of blood transfusions, phlebotomy is obviously not a realistic proposition. Repeated transfusions constitute the only effective treatment for patients suffering from chronic aplastic anemia, β-thalassemia, leukemia, refractory anemia, acquired hemolytic anemia, and sickle cell anemia. About 200 mg of iron is estimated to accumulate as a result of each unit of blood transfusion. Severe iron overload will develop after the transfusion of about 200 units of blood. Most patients die in their teens from heart or liver failure. As the body has no means of significantly increasing iron excretion, about 1 or 2 subjects in a population of 10,000 require treatment with chelating agents for safely removing toxic amounts of iron from the body.

Consequently,there is a serious need for the development of iron chelators which can be used to combat iron overload. Unfortunately, the one in common use, desferrioxamine ("Desferal", DF), is not very satisfactory, and oral administration is not currently possible due to its poor absorption from the gastrointestinal tract. It suffers also from two disadvantages. It has a very short half life in vivo, probably less than 1 hr. Also, its phenomenal binding strength depends upon its ability to wrap itself around the Fe(III) ion, and therefore it is unable to form a ternary complex with transferrin. Thus, DF has no effect on plasma ferrokinetics.

Malaria is caused by protozoan parasites belonging to the genus Plasmodium, of which the four species: P. falciparum (often fatal), P. vivax, P. malariae, and P. ovale are essentially exclusive to human beings. They have a complex life cycle in both the insect and the vertebrate host.

Malaria has undergone a resurgence over the past decade, and despite its high incidence, no revolutionary new drugs have been introduced over the past three decades. This widespread appearance of chloroquine-resistant strains of P. falciparum prompted the development of new antimalarial drugs, operating most desirably by entirely new mechanisms, to replace those to which P. falciparum has become resistant.

Iron is needed in a multitude of molecules of biological importance and participates in many vital reactions. Parasites and their host are known to compete for available iron. Both groups developed specific mechanisms to obtain this element from the surrounding medium in sufficient amount and to transport it to the appropriate

site.

The malaria parasite as any other microorganisms competes for iron even if, ironically, it lives in the erythrocyte replete with iron. It has been reported that iron supplementation in patients suffering from malaria enhance the disease, thus connecting that disease with the availability of free iron. A number of iron chelating agents have been studied related to the inhibition of P. falciparum growth in vitro.

The growth of P. falciparum in culture has been shown to be inhibited on exposure to the five major haemoglobin abnormalities: HbS (sickle cell anemia), HbH (β-thalassemia), HbF ( $\alpha_2\gamma_2$), HbE (thalassemia), and glucose-6-phosphate dehydrogenase deficiency (favism), that confer resistance to P. falciparum. The normal person has no obvious defense mechanism against invasion by the malaria parasite. Evolutionwise, the genetic alteration of normal haemoglobin appears to be the only survival means for the body to combat the malaria parasites.

It is now proposed that immunity to malaria could also be promoted by inducing a state of iron deficiency. The case in point is the resistance to malaria acquired by anaemic women during pregnancy which fades away upon administration of iron. Consistent with these lines of thought the most logical approach would be of making use of parasite metabolism for targeting the drug action at a vulnerable site of the organism. Towards this end two different approaches have been adopted.

One, making use of the susceptibility of the malaria parasite to "oxidative-stress," namely, to free oxygen radical-induced oxidative stress. Second, to affect the iron pool of the parasite controlling the biosynthesis both of metallo-protein oxidases and of the iron-dependent ribonucleotide diphosphate reductase by applying chelators inhibitory to these enzymes.

There are also ample examples in the literature indicating that iron chelators can remarkably induce regression in growth of cancerous cells in mice. The outstanding example is that of 6-formyl purine thiosemicarbazone (FPTS) which exhibits a strong anti-leukemic activity coupled with strong inhibitory effect on the iron-dependent enzyme: ribonucleotide diphosphate reductase (RdR) which plays a pivotal role in the synthesis of DNA. FPTS is highly toxic, and all efforts aimed at lowering its toxicity proved unfruitful.

The present invention is thus based on the search for iron chelators which manifest high affinity to iron both in vitro and in vivo safely and with a high efficiency when administered orally.

In an article published in J. Lab. Clin. Med. 98, 99-108 (1981) the inventors described experiments and findings with regard to "mechanism of in vivo iron chelation by pyridoxal isonicotinoyl hydrazone (PIH) and other imino derivatives of pyridoxal".

Our single crystal X-ray analysis of the complex of iron with PIH in the ratio 1Fe: 2PIH has shown that PIH functions as a tridentate ligand with $O_2N$ (two oxygen and one nitrogen atom) donor atoms. Thus, PIH reacts with iron in its tautomeric form (A), as a zwitter-ion of structure (a), as described below:

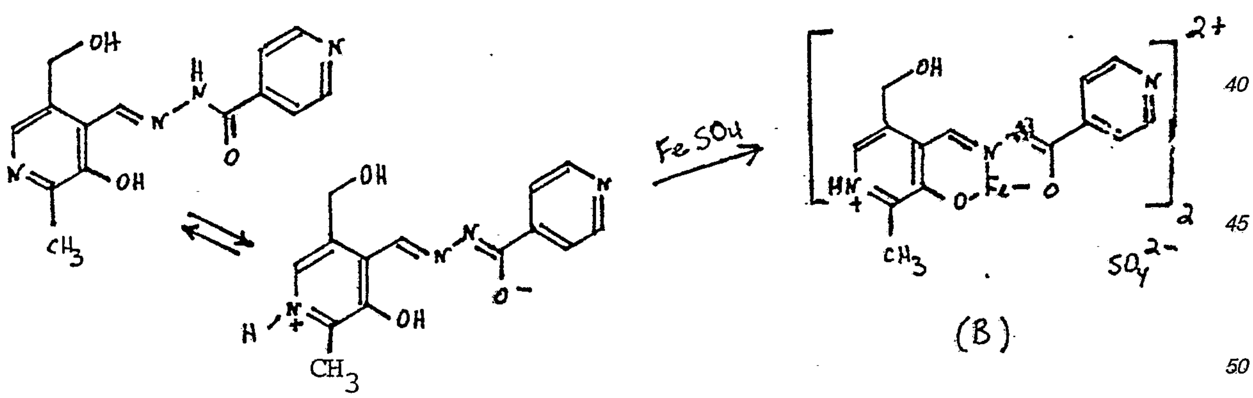


tautomeric form (A)
"zwitter-ion"

Thus, the phenolic oxygen, the carbonyl oxygen, and azomethinic nitrogen are the sites of coordination with the atoms (structure B) as evidenced by X-ray crystallography.

Structure-activity-relationship (SAR) studies indicated that the elimination of the carbonyl group and the direct linkage of the imino group to a heterocyclic ring yield hitherto unknown compounds which show remarkably significant superiority over PIH and its derivatives with respect to safety, specificity and affinity to iron, when given orally.

Thus the present invention provides a pyridoxal hydrazone derivative of the general formula I

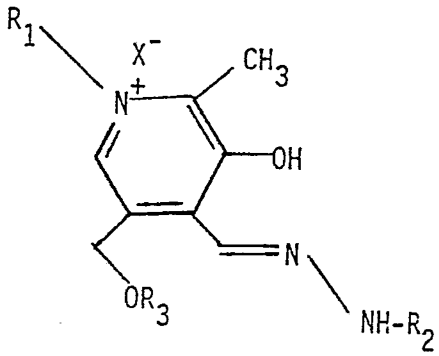

wherein
$R_1$ is an H atom or an alkyl, a methoxy-carbonylmethyl group or ethoxy-carbonylmethyl group
X is a halogen or hydroxyl group and
$R_2$ is a 4, 5, 6 or 7 membered heterocycle having one or more nitrogen, sulfur, or oxygen atoms in the heterocyclic ring, said ring being optionally substituted by one or more oxo, halogen, hydroxy, nitro, amino, cyano, trifluoromethyl, lower alkyl or alkoxy groups, and
$R_3$ is a hydrogen atom or an acetyl,propionyl or succinyl group
n is 1.

Preferred compounds of the present invention are those of the above formula wherein
$R_2$ is a 5 or 6 membered heterocycle having one or more nitrogen, sulfur, or oxygen atoms in the heterocyclic ring, said ring being optionally substituted by one or more halogen ,lower alkyl or alkoxy groups.

Especially preferred are compounds wherein $R_1$ is ethoxy carbonylmethyl or methyl, $R_2$ is selected from a pyridyl, pyrimidyl, pyrazyl, and an N-methyl pyridyl
X is I or Br, $R_1$ is H and $R_3$ is H or acetyl.

Preferably $R_3$ is H.

The compounds of the present invention can be categorized into two types with regard to their method of preparation. Type A consists of pyridoxylidene derivatives of aryl hydrazines [pyridoxal aryl hydrazones] arising from the reaction of pyridoxal with an appropriately substituted hydrazine. Type B consists of pyridoxylidenium salts, resulting from an exposure of compounds of Type A to the action of lower alkyl halides such as methyl or ethyl halides, or to the action of lower alkyl bromoacetates, such as methyl or ethyl bromoacetate. The starting materials comprise two components, one of which is always pyridoxal hydrochloride, and the other one is an appropriate hydrazino hetrocycle. The latter could be prepared by methods described in the literature. Essentially, they involve a nucleophilic substitution on an heterocyclic halide by hydrazine hydrate. The yields are highly satisfactory.

Scheme for formation of products of Type A:

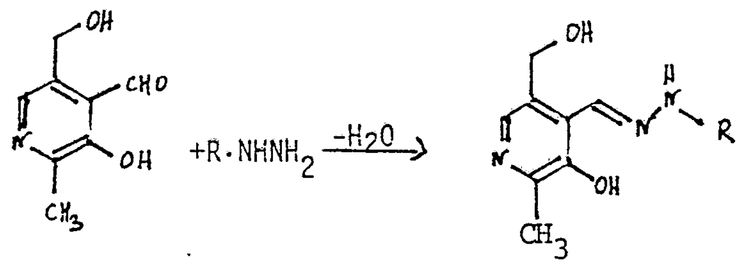


Products of Type B:

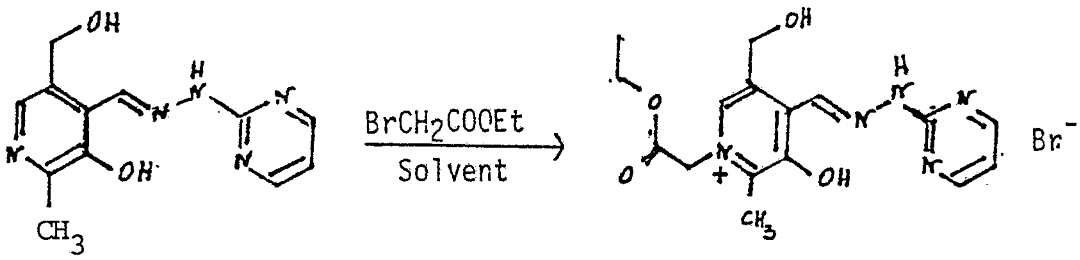


Chelators of Type B could be produced alternatively, albeit in lower yields, by forming first the pyridoxalinium salt from the reaction of pyridoxal with alkyl bromoacetate or aklyl halide, followed by its condensation with an appropriate hydrazine derivative, as delineated below:

Pyridoxal + $BrCH_2COOEt$

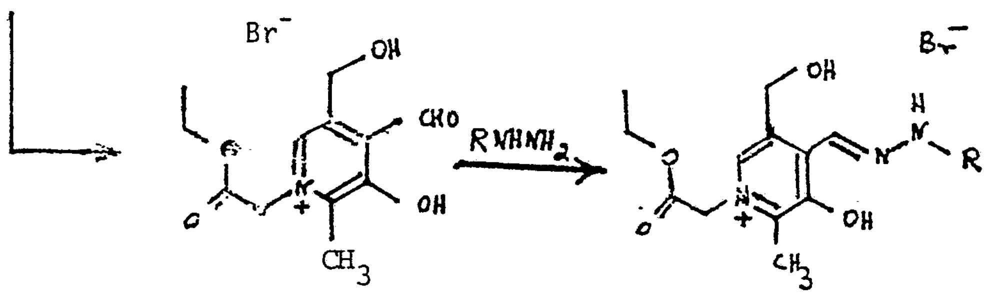


Pyridoxylinium bromide

Type B Chelator

The present invention also provides a pharmaceutical composition for the therapeutic and/or prophylactic treatment of iron overload, malaria, hepatoma, melanoma and carcinoma in mammals by in vivo iron chelation comprising as active ingredient therein a pyridoxal hydrazone derivative of the general formula I

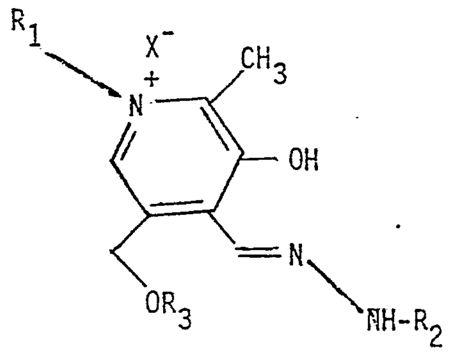

wherein
$R_1$ is an H atom or an alkyl, a methoxy-carbonylmethyl group or ethoxy-carbonylmethyl group
X is a halogen or hydroxyl group and
$R_2$ is a 4, 5, 6 or 7 membered heterocycle having one or more nitrogen, sulfur, or oxygen atoms in the heterocyclic ring, said ring being optionally substituted by one or more oxo, halogen, hydroxy, nitro, amino, cyano, trifluoromethyl, lower alkyl or alkoxy groups, and
$R_3$ is a hydrogen atom or an acetyl, propionyl or succinyl group, in combination with a pharmacologically acceptable carrier.

In preferred compositions of the present invention said 4,5,6 or 7-membered heterocycle is selected from pyridyl, furanyl, pyrrolyl, thienyl, 1,2,3-triazolyl, 1,2,4-triazolyl, imidazolyl, thiazolyl, thiadiazolyl, pyridazinyl, pyrazyl, pyrimidyl, oxazolyl, triazinyl, tetrazolyl, azetinyl, oxetanyl, thietanyl, piperidinyl, piperazinyl, imidazolidinyl, oxazolidinyl, pyrrolidinyl, tetrahydropyrimidinyl, dihydrothiazolyl, hexahydroazepinyl, purinyl, pteridinyl or one of the above groups substituted with one or more oxo, halogen, hydroxy, nitro, amino, cyano, trifluoromethyl, alkyl or alkoxy groups.

Preferably in the compositions of the present invention of the above formula, $R_2$ is a 5 or 6 membered heterocycle having one or more nitrogen, sulfur, or oxygen atoms in the heterocyclic ring, said ring being optionally substituted by one or more halogen, lower alkyl or alkoxy groups.

Especially preferred are compositions wherein said heterocyclic ring has one or more nitrogen atoms, such as
1-[pyridoxylidene]-2-[2′-pyridyl] hydrazine
1-[N-ethoxycarbonylmethyl pyridoxylidenium] -2-[2′-pyridyl] hydrazine bromide
1-[pyridoxylidene]-2-[2′-pyrimidyl] hydrazine
1 [N-ethoxycarbonyl pyridoxylidenium]-2-[2′-pyrimidyl] hydrazine
1 [N-methylpyridoxylidenium]-2-[methyl pyridinium] hydrazine hydroxide iodide
and
1-[pyridoxylidene]-2-[2′-pyrazyl] hydrazine

Thus the invention provides a composition for the prophylactic or therapeutic treatment of malaria comprising as active ingredient therein a pyridoxal hydrazone derivative of the general formula I as defined herein

The invention provides a pharmaceutical composition for treating iron overload in mammals by in vivo iron chelation comprising as active ingredient therein a pyridoxal hydrazone derivative of the general formula I as defined herein.

The invention also provides a pharmaceutical composition for the therapeutic treatment of hepatoma, melanoma and carcinoma in mammals comprising as active ingredient therein a pyridoxal hydrazone derivative of the general formula I as defined herein

The route and amount of active compound to be administered and the time of the treatment depends on the progress of the disease, the age, weight and the general condition of the patient to be treated and has to be adjusted according to the judgement of the physician.

In general amounts of from about 5 mg/kg up to 500 mg/kg, preferably of from about 10 mg/kg up to about 50 mg/kg, are administered, either parenterally or preferably orally, in a suitable conventional pharmaceutical composition.

The compounds of the formula I are of particular importance in the treatment of malaria caused by chloroquine-resistent plasmodia, in particular plasmodium falciparum.

Thus, inter alia the present invention provides a pharmaceutical or veterinary formulation comprising a pyridoxal hydrazone compound of the invention and formulated for pharmaceutical or veterinary use respectively and optionally comprising a pharmaceutically or veterinarily acceptable carrier, diluent or excipient and/or optionally being in unit dosage form.

While the invention will now be described in connection with certain preferred embodiments in the following examples so that aspects thereof may be more fully understood and appreciated, it is not intended to limit the

invention to these particular embodiments. On the contrary, it is intended to cover all alternatives, modifications and equivalents as may be included within the scope of the invention as defined by the appended claims. Thus, the following examples which include preferred embodiments will serve to illustrate the practice of this invention, it being understood that the particulars shown are by way of example and for purposes of illustrative discussion of preferred embodiments of the present invention only and are presented in the cause of providing what is believed to be the most useful and readily understood description of formulation procedures as well as of the principles and conceptual aspects of the invention.

**EXAMPLE 1:**

**1-[pyridoxylidene] 2-2′-pyridyl]-hydrazine**

(a) **Hydrochloride**.

To a strand of commercial pyridoxal hydrochloride (2g, 0.01 mole) in 5ml $H_2O$ is added dropwise a solution of 2-hydrazino pyridine (1.1g, 0.01 mole), in 15 ml 1:1-MeOH-$H_2O$ at 30-35° C. The resulting mixture is allowed to stir for an additional 2-3 hrs. and is then cooled and filtered. The yellow product (2.5g, 95%) is recrystallized from ethanol, affording yellow crystals, melting at 298°.

In the UV region of the spectrum (MeOH) it exhibits absorption bands at (mμ) 376.9 (log ε 4.307), 239.8 (4.187), 212.9 (4.207) of conc. $1.23.10^{-4}$M. At $2.46\ .10^{-4}$M conc. it gives absorption at a longer wavelength: 398 (4.146), and 370.6 (4.272), 239.1 (4.176), and 215.2 (4.141).

Its 300Mc $^1$H-NMR spectrum show resonances (DMSO) at δ 8.522 (s,1H), 8.351 ( d,1H), 8.035 (s, 1H), 7.731 (dd,1H), 6.922 (q, 2H), 4.700 (2H), 2.500 (s, 3H).

| | | | | | | | | | |
|---|---|---|---|---|---|---|---|---|---|
| Anal. | Calcd. for $Cl_2H_{15}$-$N_4O_2Cl$: | C, | 52.95; | H, | 5.09; | N, | 19.00; | Cl, | 12.03 |
| | Found: | C, | 53.12; | H, | 4.95; | N, | 18.96; | Cl, | 12.43. |

(b) Free-Base.

The free base emerged when 2-hydrazino-pyridine (0.01 mole) in MeOH-$H_2O$ was similarly reacted with pyridoxal free-base (by adding solid pyridoxal hydrochloride to 5ml aqueous 0.2m NaOH), m.p. 288°.

| | | | | | | | |
|---|---|---|---|---|---|---|---|
| Anal. | Calcd. for $C_{13}H_{14}N_4O_2$: | C, | 60.42; | H, | 5.42; | N, | 21.69. |
| | Found: | C, | 60.15; | H, | 5.33; | N, | 21.79 |

It displays at $1.25\ .10^{-4}$M conc. in MeOH UV bands at (mμ): 352.7 (4.393), 331.3 (4.332), 236.3 (4.239), 211 (4.320).

In 300Mc $^1$H-NMR spectrum (DMSO) it resonances at: δ 8.854 (s,1H), 8.235 (t,1H, J=2.4 Hz), 7.934 (s,1H), 7.720 (d,1H, J=0.75 Hz), 6.831 (t,2H, J=4.2Hz), 5.314 (s,1H), 4.603 (d,2H, J=2.7Hz), 2.413 (s,3H).

**EXAMPLE 2:**

**1-[N-methylpyridoxylidenium]-2-[methyl pyridiniumn] hydrazine hydroxido iodide**

The product of Example 1 (0.93 g, 3.5 mmoles) is dissolved in dry ethanol (15 ml) and then methyl iodide (1.2 g) added. The resulting mixture is refluxed with stirring for 24 hrs. The corresponding di-quaternary di-pyridinium salt (1.1 g 85%) precipitates out as a red solid product. It is twice recrystallized from n-amyl alcohol to yield crystals melting at 241°. In the UV region of the spectrum (MeOH) it exhibits absorption bands (mμ) at: 466.2 (5760), 399.6 (25960), 299.6 (4770), and 219.3 (29360). Its mass-spectrum exhibits peaks at m/e 272 (M-$CH_3I$ - $H_2O$), 259 (M-$CH_3I$-$CH_3OH$), 151 (259-$C_6H_8N_2$), 121 and m/e 108 ($C_6H_8N_2$, base peak).

| | | | | | | | | | | |
|---|---|---|---|---|---|---|---|---|---|---|
| Anal. | Calcd.: for $C_{15}H_{21}$-$N_4O_3I$: | C, | 41.66; | H, | 4.84; | N, | 12.96; | I, | 29.40. | |
| | Found: | C, | 41.94; | H, | 4.33; | N, | 12.95; | I, | 30.36. | M.W.432. |

**EXAMPLE 3:**

**1-[N-ethoxycarbonylmethyl pyridoxylidenium] 2-[2′-pyridyl] hydrazine bromide**

A stirred solution of the product of Example 1 (0.93 g. 3.5 mmoles) in dry ethanol (15 ml) is allowed to react with ethyl bromoacetate (1.2 gr. 7 mmoles) under reflux for 24 hours. The resulting reaction mixture is concentrated by removal of solvent to allow the corresponding yellow quaternary pyridinium salt hydrochloride to crystallize out. The supernatant fraction is diluted with ether to yield an additional portion of the desired product. The fractions (81%) are combined and recrystallized more than twice from MeOH or EtOH to yield crystals melting at 204°C.

In the UV region of the spectrum (MeOH) it exhibits a very weak band at (m μ): 651 (36), and intense bands at 407.7 (25810), 242.5 (13480) and 205.4 (15820).

The molecular-ion peak at m/e 440 could not be detected in the mass spectrum, nor a peak at m/e 285 corresponding to the ion [M-$EtOCOCH_2Br$]. However, it exhibits peaks at m/e 258, 165, 151 and a base-peak at m/e 108($C_6H_8N_2$).

| | | | | | | | | | | |
|---|---|---|---|---|---|---|---|---|---|---|
| Anal. | Calcd. for $C_{18}H_{24}N_4O_4Br$: | C, | 49.09, | H, | 5.45 | N, | 12.72, | Br, | 18.18. | |
| | Found: | C, | 48.89; | H, | 5.34; | N, | 12.16; | Br, | 18.16. | M.W. 440. |

**EXAMPLE 4:**

**1-[2′-pyrimidyl]-2′-pyridoxylidene] hydrazine (PPH)**

The title compound was prepared by allowing a solution of 2-hydrazino pyrimidine (1.32 g, 0.012 moles) in ethanol (15 ml) to react with solid pyridoxal hydrochloride (2g, 0.01 moles) which was added portionwise with stirring, followed by aqueous 0.2 NaOH (5 ml). After 5 6 hrs. of stirring at r.t., the yellow precipitate was collected (2.29 g, 88%), and recrystallized from ethanol, mp. 298°. The free base exhibited UV absorption bands (MeOH) at (A°): 3425, 3398, 3074, 2322 and 2055, its 300Mc $^1$H-NMR spectrum (DMSO-$d_6$) showed resonances (δ) at 12.557 (1H). 12.020 (1H), 8.782 (1H), 8.665 (2H), 8.01 (1H), 7.736 (1H), 5.441 (1H), 4.68 (2H), 2.50 (3H). It exhibits the molecular-ion peak at m/e 259.

| | | | | | | | |
|---|---|---|---|---|---|---|---|
| Anal. | Caldc. for $C_{12}H_{13}N_5O_2$: | C, | 55.59; | H, | 5.02; | N, | 27.02 |
| | Found: | C, | 55.56; | H, | 5.03; | N, | 26.76 |

**EXAMPLE 5:**

**1-(N-ethoxycarbonyl pyridoxylidenium]-2-[2′-pyrimidyl] hydrazine**

The product of Example 1 (10.4 g, 40 mmoles) is mixed with ethyl bromoacetate (20g, 0.12 moles) and dry ethanol (700 ml) and allowed to reflux with stirring for 48 hrs. The desired product (16.2g ,95%) crystallized out towards the end of the reaction. Recrystallization from dry ethanol afforded yellow crystals, mp 188°C.

In the mass spectrum it displays the parent molecular ion peak of 1-[N-pyridoxylidene]-2-[2′-pyridyl] hydrazine at m/e 259 (M-$CH_2COOEt$). and peaks at m/e 165, 150, 123 arising from further losses of $CH_3$ and ($CH_3$+OH) groups, respectively. The base-peaks at m/e 110 and 108 are attributable to $[C_4H_6N_4]^+$ and $[C_4H_4N_4]^+$ ions assumed to correspond to the pyrimidyl hydrazine portion of the molecule.

In the UV region of the spectrum (MeOH) it displays absorptions at (mμ): 383.1 (17660), 379.6 (17490), 239.4 (13200), 214.6 (11280) and 206.1 (11680). In the infrared region of the spectrum (KBr) it shows bands (cm$^{-1}$) at: 3280, 3150, 1740 (ester), 1638 (amide I), 1565, 1530 (amide II).

| | | | | | | | | | | |
|---|---|---|---|---|---|---|---|---|---|---|
| Anal. | Calcd. for $C_{16}H_{22}$-$N_5O_4Br$: | C, | 43.24, | H, | 4.95; | N, | 15.76; | Br, | 18.02 | |
| | Found: | | 43.49; | H, | 4.68; | N, | 15.83; | Br, | 17.80. | M.W. 444. |

**EXAMPLE 6:**

**1-[2′-pyrazyl]-2-[pyridoxylidene] hydrazine**

1-[2′-pyrazyl]-2-[pyridoxylidene] hydrazine was prepared in a fashion described for 1-[2′-pyrimidyl]-2-[pyridoxylidene] hydrazine (Ex. 4) by reacting 2-hydrazinopyrazine with pyridoxal in ethanol-$H_2O$ solution to form the respective hydrazone (80% yield) mp 288-290°

| | | | | | | | |
|---|---|---|---|---|---|---|---|
| Anal. | Cald. for $C_{12}H_{13}N_5O_2$: | C, | 55.59; | H, | 5.02; | N, | 27.02 |
| | Found: | C, | 55.36; | H, | 5.10,; | N, | 26.74 |

**EXAMPLE 7:**

**COPPER(II) Chelate of 1-pyridoxylidene]-2-[2′-pyridyl] hydrazine sulphate**

When an aqueous solution of 1-[pyridoxylidene]-2-[2′-pyridyl] hydrazine hydrochloride (2g 6.78 mmoles) in 5 ml $H_2O$ is mixed with $Cu(II)SO_4.5H_2O$ (0.85g, 3.39 mmoles) in 5 ml $H_2O$, a dark brown precipitate emerges, which after washings with water and ethanol analyzed as a $C_{26}H_{28}N_8Scu$ coordination compound.

Its UV spectrum exhibits absorption bands at (mμ) 466.7(logε ), 409.6 (4.16), 338.4 (4.23), 238.2 (4.49).

**COMPARATIVE EXAMPLE A:**

**1-[pyridoxylidene]-2-[N-methylisonicotinoylium] hydrazine iodide**

Solid pyridoxal hydrochloride (2g., 0.01 moles) is added to aqueous 0.2M NaOH (5 ml), followed by an addition of an aqueous solution (15 ml) containing isoniazide methoiodide (2.8 g, 0.012 moles). The resulting mixture is stirred for 5-6 hrs at room temperature, and the orange precipitate collected (3.3 g, 80%). The corresponding hydrazone thus formed is recrystallized twice from ethanol to yield yellow-orange needles melting at 250°C. In the UV region of the spectrum (MeOH) it exhibits bonds (mμ): 342.2 (16400), 302.9 (23580), 218.4 (38800), IR (KBr): 3520, 3380, 1670 (amide I), 1530, 1472 $cm^{-1}$ amide II). In the mass spectrums (EI) it exhibits a peak at m/e 285 ($M-CH_3I$) and peaks at m/e 164 ($C_8H_8N_2O_2$) and 106 ($C_6H_4NO$, base-peak).

| | | | | | | | | | | |
|---|---|---|---|---|---|---|---|---|---|---|
| Anal. | Calcd. for $C_{15}H_{17}$-$H_4O_3I$: | C, | 42.96; | H | 3.97; | N, | 13.08; | I, | 29.67. | |
| | Found: | C, | 42.05; | H, | 4.05; | N, | 13.03; | I | 29.43. | M.W.428. |

Table 1 describes the fate of $^{59}Fe$ tracer reincubated in vivo with 10 mg of some of the above iron chelators and then injected subcutaneously to groups of 4 normal rats. Numbers represent mean cumulative radioiron excretion within 4 days of injection. For comparison, spontaneous excretion of radioiron and the excretion of desferrioxamine (DF) bound radioiron are also given. The chelators of the present invention are also compared with PIH and with the related compound from comparative Example A.

As shown in Table 1, the compounds of the present invention are able to enhance the excretion of radioiron above that of untreated controls, and several showed considerable radioiron excretion activity comparable to and even exceeding that of DF and were more than three times as effective as PIH and the compound from comparative Example A.

TABLE 1

Radioiron Distribution in %

| Code number | Blood | Liver | Spleen | Urine | Feces | Total excretion |
|---|---|---|---|---|---|---|
| Control | 27.4±1.6 | 12.1±0.6 | 0.47±0.03 | 0.1±0 | 3.1±0 | 3.2±0 |
| Ex. 2 | 13.3±2.3 | 6.8±0.4 | 0.51±0.02 | 39.0±3.1 | 27.0±2.2 | 66.0±5.3 |
| Ex. 3 | 6.4±0.4 | 3.3±0.4 | 0.16±0.04 | 29.5±0.6 | 57.2±1.1 | 86.7±1.7 |
| Ex. 5 | 4.2±0.2 | 2.9±0.2 | 0.06±0.03 | 28.9±1.0 | 67.3±2.4 | 96.2±3.4 |
| Desferral | 8.7±1.6 | 2.7±0.3 | 0.14±0.03 | 71.9±3.6 | 13.5±0.7 | 85.4±4.3 |
| Ex. A | 33.3±2.1 | 12.8±0.2 | 0.56±0.02 | 3.7±0.2 | 22.7±1.1 | 26.4±1.3 |
| * | 25.1±2.2 | 8.2±0.8 | 0.36±0.03 | 30.6±1.12 | 8.6±0.3 | 39.2±1.6 |

* Comparative Compound B: 1-[N-methyl pyridoxylidenium]-2-[N′-isonicotinoylium] hydrazine dimethiodide

It has thus been found that unlike PIH in which the ring nitrogens do not bind the metal, in e.g., 1-[2′-pyridyl]- and 1-[2′-pyrimidyl]-2-[pyridoxylidene] hydrazines both the pyridyl and pyrimidyl ring nitrogens do bind to iron and are therefore the sites of coordination to the metal as shown in the followng structure.

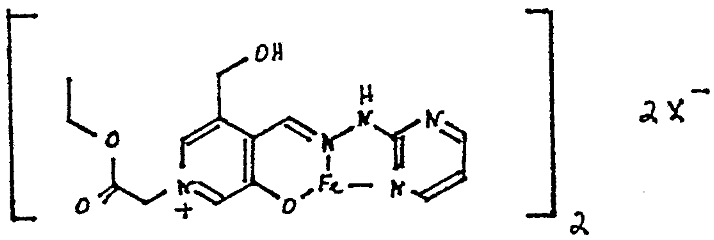

It is therefore assumed that the participation of the ring heteroatom of the novel compounds of the present invention as a binding ligand function as an enhancer both with respect to specificity and affinity to iron, rendering to it the superior properties exhibited thereby.

Table 2 describes the ability of the preferred compounds of examples 2 and 3 to mobilize iron from hepatic parenchymal stores in hypertransfused rats labelled by the intravenous injection of $^{59}$Fe-ferritin. In untreated control animals 91% of the injected radioactivity is retained in the hepatic parenchyma and only 3% are reutilized for the production of newly formed erythrocytes. Two to three % of the radioactivity is found in other (residual) organs or excreted spontaneously in the stool. In contrast, in treated animals there was a significant reduction in radioactivity and a reciprocal increase in fecal radioiron excretion. In each case, 40 mg of each compound was administered per animal in a single subcutaneous injection.

TABLE 2

Ferritin-$^{59}$Fe distribution in hypertransfused rats in %

| Compound | (n) | Blood | Liver | Spleen | Residual | Urine | Feces |
|---|---|---|---|---|---|---|---|
| Control | (4) | 3.0±0.1 | 90.6±12.3 | 0.5±0.1 | 2.3±0.1 | 0.3±0 | 3.3±0.1 |
| Desferral | (4) | 4.5±0.4 | 70.4±6.0 | 0.6±0.1 | 0 | 1.2±0 | 23.3±0.1 |
| Ex.2 | (4) | 3.0±0.1 | 35.5±1.7 | 0.4±0.1 | 0 | 2.2±0.1 | 58.9±3.0 |
| Ex. 3 | (4) | 3.8±0.2 | 36.5±1.2 | 0.4±0.1 | 0 | 1.7±0.1 | 57.6±2.9 |

BIOLOGICAL STUDIES

In vivo studies of chelating efficiency were performed in two models: (a) normal rats injected i.p. with 10 mg of either desferrioxamine B (DF), PIH of the prior art, PPH of Example 4 or PPHE from Example 5 labelled in vitro with $^{59}$Fe; and (b) hypertransfused rats receiving twice 4 ml/100gm packed RBC intravenously 5 and 3 days prior to study (hematocrit 68%) and labelled with $^{59}$Fe-ferritin injected i.v. 24h prior to iron chelating therapy as described previously. Drugs to be tested in the $^{59}$Fe-ferritin model were given either by i.p. injection or by gastric tube at a dose of 40 mg per animal. Radioiron excretion and organ distribution were determined after 4 days

The radioactivity of spleen, weighed portions of the liver, and 1 ml samples of blood were determined in an automatic well type scintillation counter (Packard Autogamma Model 5360 Scintillation spectrometer). Spleen and kidney were counted as whole organs.

Whole body counts were performed in a small animal counter (Packard Model 446 Armac) scintillation detector. The excretion of radioiron following $^{59}$Fe labelling was measured in animals confined to solitary metabolic cages with stainless steel grid bottoms, and stool and urine were collected separately.

RESULTS AND DISCUSSION

From Table 3 it is evident that both PPH and PPHE function as tridentate ligands manifesting higher affinity ($\sim$30 fold) for ferrous than for ferric ions. Seemingly placing an ethoxycarbonylmethyl group at the pyridinic ring nitrogen (PPH>PPHE) affects most notably the affinity of the ligand for cupric ions. This affinity diminishes from $K = 9x10^9$ (in PPH) to $2x10^4$ (in PPHE), whereas that for iron on the contrary increases, respectively, from $3x10^9$ to $1x10^{10}$. Most significantly, it affects the $K_{Fe}/K_{Cu}$ ratio ($= 4x10^5$), rendering (PPHE) to be highly specific for iron. This long range N-substitution effect suggests electronic interaction through $\pi$-bonds between the metal and the pyridoxal moiety in the chelate, since the pyridoxal ring-nitrogen is not involved in metal chelation. Only the phenolic hydroxyl group ($H^c$), the hydrazinic $sp^2$ nitrogen and the pyrimidinic ring nitrogen participates in metal binding. It will be noted that in this test PPHE of the present invention was about sixty times more effective than the prior art PIH.

Significantly the mass spectrum (EI) of PPHE exhibit a peak at m/z 259, corresponding to a loss of $BrCH_2COOEt$ (EBA) from the molecular-ion [PPHE > PPH], in harmony with the assigned pyridinium salt

structure.

TABLE 3

CHELATION OF (I) AND (II) WITH FERROUS AND CUPRIC IONS IN $H_2O$ AT $10^{-4}$M CONCENTRATION AND pH 6.50 ± 0.15

| LIGAND | COPPER CHELATION RATIO Cu:L | mμ | $\varepsilon x10^3$ | K apparent | IRON CHELATION RATIO Fe:L | mμ | $\varepsilon x10^3$ | K apparent |
|---|---|---|---|---|---|---|---|---|
| DESFER-RIOX-AMINE | - | - | - | - | 1:1 | 402 | 18 | $4.5x10^3$ |
| PIH | 1:1 | 405 | 14 | $2.3x10^4$ | 1:2 | 453 | 16 | $3.8x10^8$ |
| Ex.4 | 1:2 | 443 | 12.8 | $9x10^{10}$ | 1:2 | 420 | 2.15 | $3x10^9$ |
| Ex.4* | - | - | - | - | 1:2 | | | $1x10^8$ |
| Ex.5 | 1:1 | 460 | 70 | $2x10^4$ | 1:2 | 439 | 42 | $1x10^{10}$ |

(*) With ferric ions at comparable conditions

Hypertransfused rate have been employed in which hepatocellular iron stores were labelled selectively by the intravenous injection of $^{59}$Fe-ferritin (Table 4). As shown in Table 4 ,40 mg of desferral resulted in the excretion of 23% of the injected radioactivity in the stool, representing biliary excretion. Urinary excretion was negligible. The preferred compounds of the present invention resulted in the excretion of nearly 3 times as much radioiron than an identical dose of desferral.

Finally, the effectiveness of oral treatment with these compounds was compared with the effect of oral desferral. Following the administration of 40 mg drug/animal by gastric tube to hypertransfused $^{59}$Fe-ferritin labelled rats, total radioiron excretion in untreated controls was 4.6%, in DF-treated animals 6.0%, and with these compounds 9.4% and 17.6% respectively. This represents, in the case of the compound of example 3 a 31% efficiency as compared to the parenteral administration of the same drug.

These results represent a very significant progress in providing new compounds of potential clinical usefulness in the management of patients with transfusinoal iron overload. The compound of example 3 is 3 times as effective as DF on a weight per weight basis, has a selective interaction with parenchymal cells, and satisfactory oral effectiveness.

In untreated controls 91% of the injected radioactivity has been retained in the liver and spontaneous radioiron excretion within 4 days was 3.6%. The injection of 40 mg DF i.p. resulted in a 20% reduction in hepatocellular radioactivity and an almost identical increase in fecal radioiron excretion. By comparison, 40 mg PPHE i.p. resulted in a 69% reduction in hepatic radioactivity and the excretion of 72% of the radioiron label in the stool. Finally, the oral administration of 40 mg PPHE resulted in a 27% reduction in hepatic $^{59}$Fe and the excretion of 28%, i.e. a roughly 20% greater oral chelating efficiency than an identical dose of parenteral DF.In contradistinction it is known that Desferral has no oral efficiency.

TABLE 4

ORGAN DISTRIBUTION AND EXCRETION OF $^{59}$Fe-FERRITIN IN HYPERTRANSFUSED RATES (%)

| CONDITION | (n) | BLOOD | LIVER | URINE | STOOL | TOTAL EXCRETION |
|---|---|---|---|---|---|---|
| CONTROL | (4) | 3.0±0.1* | 90.6±12.3 | 0.3±0 | 3.3±0.1 | 3.6±0.1 |
| DESFERRAL i.p. | (4) | 4.5±0.4 | 70.4± 6.0 | 1.2±0 | 23.3±0.1 | 24.5±0.1 |
| Ex. 4 | (4) | 4.5±1.0 | 21.6± 6.5 | 0.5±0.1 | 71.5±2.5 | 72.0±2.6 |
| Ex. 5 | (4) | 3.1±0.6 | 63.8± 8.7 | 0.5±0 | 27.6±1.3 | 28.1±1.3 |

* mean ± ISD

Preliminary toxicity studies performed at our laboratory in rats have shown that at the effective therapeutic doses of 100 mg/kg/d by oral or parenteral administration, PPHE is well tolerated. Detailed biochemical tests of hepatic and renal function and complete blood counts as well as hepatic and renal histology revealed no indications of toxicity after 3 months of continuous treatment.

In spite of the increasing body of evidence supporting the clinical efficacy of desferrioxamine (DF), the only iron chelating medication available at present for clinical use, a number of serious limitations remain, underlining the need for the development of other, improved iron chelating medications . These limitations

include the need for parenteral administration caused by the inefficient intestinal absorption of DF; its almost prohibitive price limiting its use to affluent populations and; its short duration of action caused by a combination of rapid clearance and enzymatic breakdown . New inexpensive, and orally effective iron chelators would not only make long-term chelating therapy more acceptable, but would increase its therapeutic efficacy by virtue of an uninterrupted supply of circulation drug absorbed from slowrelease oral preparations. Experience gained in the management of thalassemic patients and in experimental models of immune complex vasculitis, islet cell allotransplantation and paraquat toxicity has shown that it is not only the chelating power, but the uninterrupted supply of a chelating drug and its ability to penetrate critical extracellular and intracellular iron compartments, that determines its ability to modify disease. The development of PPHE and of the related compounds described and claimed herein and especially their identification as effective oral iron chelating drugs, is a significant step towards the realisation of these objectives.

Cultures of a chloroquine-resistant strain of P. falciparum (FCR-3), cultivated in human erythrocytes (a technique widely used at the Kuvin Centre, Hebrew University Hadassah Medical School), were suspended in solutions containing chelators and then cultured for 3 days. Growth of parasites was determined by either slide counts or by incorporation of $^3$H-hypoxanthine. The parasite growth was monitored by microscopic slide counts. The effects of compounds according to the present invention on the growth of P. falciparum chloroquine-resistant strains are demonstrated in Table 5 which summarizes the data on inhibitory effects of some chelators (Ex. 1, 3, 5) and one copper chelate (Ex. 7) on the malaria parasites.

TABLE 5

INHIBITORY EFFECTS OF SOME CHELATORS ON THE GROWTH OF P. falciparum

| CODE NUMBER | TOTAL EXCRETION OF RADIO-IRON IN RATS % | INHIBITION OF GROWTH OF DRUG-RESISTANT STRAIN OF P. falciparum | |
|---|---|---|---|
| | | % INHIBITION | MOLAR CONCENTRATION |
| Control | 3.2 | 0 | -- |
| Desferrioxamine | 85.4± 4.3 | complete after 96 Hrs. (*) | 1.5 x $10^{-5}$ |
| Ex. 1 | 34.2 | 93.4 | 2 x $10^{-6}$ |
| Ex. 3 | 86.7± 1.7 | 90.9 | 2 x $10^{-6}$ |
| Ex. 5 | 96.2± 3.4 | 90.3 | 2 x $10^{-5}$ |
| Ex. 7 | --- | 100 (**) | 1 x $10^{-12}$ |
| Ex. A | 26.4± 1.3 | 16.6 | 2 x $10^{-5}$ |

(*) With non-resistant strain of P. falciparum according to Raventos-Suarez.
(**) After 2 hours of exposure.

From Table 5 it can be seen that the human malaria parasite, Plasmodium falciparum is sensitive to lipophilic chelators based on pyridoxal. Its sensitivity is particularly high towards the copper chelate of pyridoxal 2-pyridyl hydrazone (EX. 7) at concentrations as low as 1 x $10^{-12}$M, making them considerably more potent than quinine sulfate in the same system. Growth inhibition of the parasite does not seem to correlate directly with the ability of the compound to mobilize iron from the body (compare EX. 1 with EX. 3), or with its metal binding constants. On the other hand, the antimalarial effects are potentiated by iron, and most significantly antagonized by anti-oxidants and desferrioxamine. These suggest that the pyridoxal based chelators function most likely as electron-transfer oxidants, promoting iron-catalyzed Fenton-type reaction that leads to enhanced oxidative stress.

Further tests of the compounds of the present invention have shown that they were able to inhibit the growth of a rat hepatoma, and human melanoma, bladder carcinoma and lung epithelial carcinoma cells lines, at concentrations down to 1 µg of chelator per 1 ml of growth media (4.$10^{-6}$M).

In line with these results we become interested in assessing the ability of the new chelators of the present invention to inhibit the growth of P 388 cells (mice leukemia) together with their ability to inhibit the enzymatic activity of RdR (inhibition of DNA synthesis). It becomes possible also to look at the ability of one of the compounds (Ex. 1) to inhibit the growth of Lewis lung carcinoma. With a view to possible mechanisms we

employed the well-established anti-cancer antibiotics (a) bleomycin, and (b) daunorubicin, as reference compounds. The results obtained are summarized in Tables 6 and 7.

From Table 6 it can be seen that compounds: Ex. 1, 4 and 6 inhibit the activity of ribonucleotide diphosphate reductase (RdR) in the order 6> 1> 4. Most striking is the effect of alkylation at the ring-nitrogen of the pyridoxylidene moiety by ethyl bromoacetate (Ex. 3) which entails obliteration of the anti-RdR activity shown by compound Ex. 1. This has direct bearing on the superiority of compounds such as Ex. 3 and Ex. 5 as safe drugs for removal of toxic accumulation of iron.

TABLE 6

| | **DOSE** | | | Inhibition of Ribonucleoside Diphosphate Reductase (RdR)(*) % | Inhibition of Lewis Carcinoma(*) | |
|---|---|---|---|---|---|---|
| | $M10^{-5}$ | µg/ml | mg/kg | | Primary Tumor % | Metastases % |
| Bleomycin | - | - | 1.25 | - | 24 | 48 |
| Hydroxyurea | 100 | 76 | - | 73 | - | - |
| | 10 | 7.6 | - | 37 | | |
| | 1 | 0.8 | - | 15 | | |
| Ex. 1 | 6.7 | 20 | - | 72 | - | - |
| | 5.0 | - | 25 | - | 0 | 41 |
| Ex. 3 | 10 | 40 | - | none | - | - |
| Ex. 4 | 100 | 260 | - | 78 | - | - |
| | 10 | 26 | - | 40 | | |
| | 1 | 2.6 | - | 30 | | |
| Ex. 6 | 100 | 260 | - | 83 | - | - |
| | 10 | 26 | - | 81 | | |
| | 1 | 2.6 | - | 64 | | |

TABLE 7

Tumor: Leukemia P 388 Treatment Schedule: days 1, 2, 3, 4

Graft: i.p., $10^6$ cells Mice: female $B_6D_2F_1$

Route of Administration: i.p. Duration of Observation: 24 days

Preparation of Suspension: Tween 80 + distilled water

| Compound Tested | Dose (mg/kg) | Bodyweights Day 7-Day (g) | Activity* T/C x 100 | Survivors on Day 20 |
|---|---|---|---|---|
| Daunorubicin [Reference] | 1 | -1.9 | 154 | 0/10 |
| Ex. 1 | 75 | -0.6 | Toxic(xx) | 0/5 |
| | 50 | +0.8 | 126(xxx) | 0/5 |
| | 37.5 | +0.2 | 110 | 0/5 |
| | 25 | +2.6 | 105 | 0/5 |

(X) TC/ X 100 = $\frac{\text{mean survival time for treated mice}}{\text{mean survival time for controls}}$

(xx) 3d/5 after 2 treatments

(xxx) P<0.05 (Mann-Whitney Test)

It will be evident to those skilled in the art that the invention is not limited to the details of the foregoing illustrative examples and that the present invention may be embodied in other specific forms without departing from the essential attributes thereof, and it is therefore desired that the present embodiments and examples be considered in all respects as illustrative and not restrictive, reference being made to the appended claims, rather than to the foregoing description, and all changes which come within the meaning and range of equivalency of the claims are therefore intended to be embraced therein.

## Claims

1. A pyridoxa] hydrazone derivative of the general formula I

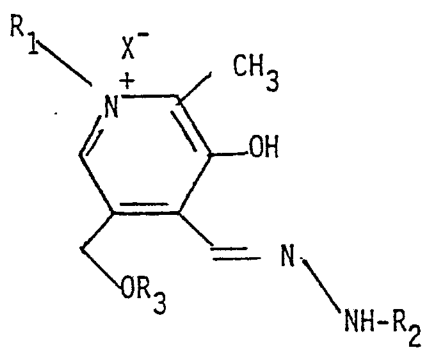

wherein
$R_1$ is an H atom or an alkyl, a methoxy-carbonylmethyl group or ethoxy-carbonylmethyl group
X is a halogen or hydroxyl group and
$R_2$ is a 4, 5, 6 or 7 membered heterocycle having one or more nitrogen, sulfur, or oxygen atoms in the heterocyclic ring, said ring being optionally substituted by one or more oxo, halogen, hydroxy, nitro, amino, cyano, trifluoromethyl, lower alkyl or alkoxy groups, and $R_3$ is a hydrogen atom or an acetyl,propionyl or succinyl group.

2. A pyridoxal hydrazone derivative as claimed in claim 1 wherein $R_2$ is a 5 or 6 membered heterocycle having one or more nitrogen, sulfur, or oxygen atoms in the heterocyclic ring, said ring being optionally substituted by one or more halogen, lower alkyl or alkoxy groups.

3. A pyridoxal hydrazone derivative as claimed in claim 1 or claim 2 wherein $R_2$ is a pyridyl, pyrimidyl, pyrazyl, or an N-methyl pyridyl group; and/or wherein X is $I^-$ or $Br^-$; and/or wherein $R_1$ is ethoxy-carbonylmethyl, methyl or ethyl; and/or wherein $R_3$ is H or acetyl.

4. A pyridoxal hydrazone as claimed in claim 1 wherein $R_2$ is a 4,5,6 or 7-membered heterocycle selected from pyridyl, furanyl, pyrrolyl, thienyl, 1,2,3-triazolyl, 1,2,4-triazolyl, imidazolyl, thiazolyl, thiadiazolyl, pyridazyl, pyrazyl, pyrimidyl, oxazolyl, triazinyl, tetrazolyl, azetinyl, oxetanyl, thietanyl, piperidyl, piperazyl, imidazolidyl, oxazolidyl, pyrrolidyl, tetrahydropyrimidyl, dihydrothiazolyl, hexahydroazepyl, puryl, pteridyl or one of the above groups substitued with one or more oxo, halogen, hydroxy, nitro, amino, cyano, trifluoromethyl, alkyl or alkoxy groups.

5. A pyridoxal hydrazone as claimed in claim 1 which is 1-[pyridoxylidene]-2-[2′-pyridyl] hydrazine; 1-[N-ethoxycarbonylmethyl pyridoxylidenium] -2-[2′-pyridyl] hydrazine bromide; 1-[pyridoxylidene]-2-[2′-pyrimidyl] hydrazine; 1-[N-ethoxycarbonyl pyridoxylidenium]-2-[2′-pyrimidyl] hydrazine; 1-[N-methylpyridoxylidenium]-2-[methyl pyridinium] hydrazine hydroxide; 1-[N-ethoxycarbonylmethyl pyridoxylidenium]-2-[2′-pyrimidyl] hydrazine bromide; or 1-[pyridoxylidene]-2-[2′-pyrazyl] hydrazine.

6. A process for the production of a pyridoxal hydrazone as defined in claim 1 comprising either

(a) reacting a compound having the formula

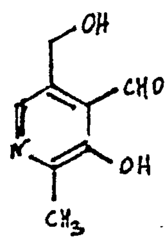

with a compound having the formula R $NHNH_2$;

(b) reacting a compound having the formula

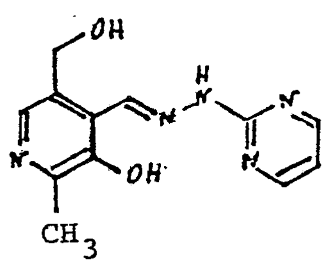

with a compound having the formula $BrCH_2COOEt$; or

(c) reacting a compound of the formula

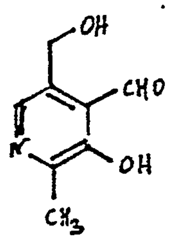

with a compound of the formula

$BrCH_2COOEt$
and reacting the product with a compound of the formula

$RNHNH_2$.

7. A pharmaceutical or veterinary formulation comprising a pyridoxal hydrazone as defined in any one of claims 1 to 5 and formulated for pharmaceutical or veterinary use respectively, and optionally comprising a pharmaceutically or veterinarily acceptable carrier, diluent or excipient and/or optionally being in unit dosage form.

8. A pharamaceutical composition for use in the treatment and/or prophylaxis of
(a) iron overload;
(b) malaria; or
(c) hepatoma, melanoma and carcinoma
comprising a pyridoxal hydrazone as defined in any one of claims 1 to 5, together with a pharmacologically acceptable carrier.

9. The use of a pyridoxal hydrazone as defined in any one of claims 1 to 5 in the manufacture of a medicament.

10. The use as claimed in claim 9 wherein the medicament is packaged together with instructions for its use in the treatment and/or prophylaxis of
(a) iron overload;
(b) malaria; or
(c) hepatoma, melanoma and carcinoma.